# EUROPEAN PATENT SPECIFICATION

(11) **EP 1 516 585 B1**
(45) Date of publication and mention of the grant of the patent: **06.06.2012**
(21) Application number: 04021532.9
(22) Date of filing: 10.09.2004
(51) Int. Cl.: A61B 5/15

(54) **Non-evacuated blood collection tube**
Nicht-evakuiertes Blutentnahmeröhrchen
Tube de prélèvement de sang non-évacué

(30) Priority: 22.09.2003 US 667920
(43) Date of publication of application: 23.03.2005
(73) Proprietor: Becton, Dickinson and Company, Franklin Lakes, New Jersey 07417 (US)
(72) Inventor: Manoussakis, Dimitrios, Wyckoff NJ 07481 (US)
(74) Representative: von Kreisler Selting Werner

(56) References cited:
- DE-A- 2 706 566
- US-A- 4 572 210
- US-A- 4 646 753
- US-A- 4 805 635
- US-A- 5 165 419
- US-A- 5 269 317

## Description

### BACKGROUND OF THE INVENTION

### Field of the Invention

The present invention relates to a blood collection tube. More particularly, the present invention relates to a non-evacuated blood collection tube for use by medical professionals in the collection of fluid samples from humans.

### Description of Related Art

Blood collection containers are well-known in the medical arts. They are used to store a sample of blood obtained by a phlebotomist from a patient until the blood is ready to be tested or used for other purposes. Currently, most blood collection containers on the market are evacuated. That is, a vacuum exists within the interior of a collection tube and a piercible stopper is located at the injection end of the collection tube. The vacuum is used to provide a draw on the blood received from a patient into the interior of the collection tube. In an ideal situation, once the piercible stopper end of a typical blood collection tube is pierced via a needle in a blood collection set, blood will be drawn into the interior of the collection tube until the vacuum is exhausted, or more likely, until the tube is removed from the needle of the blood collection set.

Ideal situations in the practice of blood collection do not always occur. Evacuated blood collection tubes need to maintain the vacuum inside the collection tube in order for the blood sampling to work. Once the vacuum is gone, the tube will no longer function as promised. Moreover, it is difficult to control the rate of draw by means of an evacuated tube, other than with partial draw tubes. This uncontrollability can give rise to vein collapse if the vacuum draw is too strong for a given patient's veins, especially a concern in the elderly and infants or severely ill patients.

Blood extraction devices for small sample volumes within sample tubules has been proposed, for example in U.S. Patent No. 5,115,817 to Sarstedt. This patent discloses a sample tubule including an inner tube therein which is closed through a cap, and which includes a hermetic seal provided through a self-closing membrane. During blood extraction, a needle pierces through the membrane and blood is collected within the inner tube. After extraction, the cap is unscrewed, and air vents within the sample tubule vent air such that the inner tube can be removed from the sample tubule while the blood is conveyed at the bottom of the sample tubule. Such a blood extraction device does not provide for displacement of air within the sample tubule during the extraction, and therefore provides for limited sampling capability. Also, the device of this patent includes open vent bores for transfer of the blood into the sample tubule which pose a risk for contamination, and requires a complicated procedure for removal of the inner tube to transfer the blood to the sample tubule.

A specimen collection container of the type defined in the introductory part of claim 1 is disclosed in DE 27 06 566 A1. This container is a blood collection container comprising a non-evacuated collection tube, which is closed on one end by a pierceable stopper and on the other end by a gel-like substance having a specific weight between the specific weight of the lighter phase and the heavier phase of the blood sample. A venting hole is provided in the sidewall of the tube. Thus, air may escape from the tube to the environment when a blood sample is taken by using a cannula having one end positioned within the chamber of the tube.

In U.S. 4,572,210 A, a syringe device is described for obtaining a gas-free blood sample. The syringe device has a tubular body comprising a sealing member in the form of an elastomeric stopper. The stopper comprises a passageway extending in axial direction therethrough. The passageway leads into a hollow plunger rod. The hydrophobic filter membrane is disposed across the front face of the sealing member. The membrane establishes passageway sealing means to seal the vent or air flow passage from a blood collection chamber through the plunger rod to the outside.

### SUMMARY OF THE INVENTION

It is an object of the invention to provide a non-evacuated blood collection tube for use is medical sampling procedures, which is reliable, safe, convenient, and cost-effective.

The specimen collection container according to the present invention is defined by claim 1.

The present invention is directed to a non-evacuated specimen collection container which is capable of collecting a blood sample from a patient based on the physiological venous pressure of the patient, without the need for any internal vacuum within the collection container. The collection container includes a non-evacuated collection tube having a tubular wall extending between an open top end and a bottom end defining an interior chamber, a piercible closure sealing the open top end of the collection tube, and a vent adapted for displacement of air from within the interior chamber of the collection tube to an exterior of the collection tube during collection of a liquid sample within the interior chamber of the collection tube. The vent is adapted for maintaining the interior chamber at ambient pressure prior to collection of a sample, and may be a two-way vent. The vent is adapted to seal upon contact with the liquid sample so as to prevent further displacement of air into or out from the tube.

The tube is open at both the top and bottom ends, with a piercible stopper sealing the top end and a hybrid stopper including a venting filter sealing the bottom end. In a further embodiment, a collapsible bag may be removably mated with the open top end of the collection tube, with the bag positioned in the interior chamber of the tube. In this manner, the blood sample may be collected within the collapsible bag, with the bag expanding upon filling under venous pressure.

### DESCRIPTION OF THE DRAWINGS

FIG. 1 is a perspective view of a collection tube in accordance with the present invention;

FIGS. 2 and 3 are exploded perspective views of the collection tube of FIG. 1;

FIG. 4 is a side cross-sectional view of the collection tube of FIG. 1;

FIG. 5 is a perspective view of the collection tube of the present invention in use with a needle during a blood collection procedure;

FIG. 6 is a top perspective view of a collection tube in accordance with a further embodiment of the present invention;

FIG. 7 is a bottom perspective view of the collection tube of FIG. 6;

FIG. 8 is a side cross-sectional view of the collection tube of FIG. 6;

FIG. 9 is an exploded perspective view of a collection tube in a further embodiment;

FIG. 10 is a side cross-sectional view of the tube of FIG. 9;

FIG. 11 is a perspective view of a further embodiment in an empty state prior to collection of a sample;

FIG. 12 is a perspective view of the tube of FIG. 11 filled with a sample;

FIG. 13 is a side cross-sectional view of the tube of FIG. 11; and

FIG. 14 is a side cross-sectional view of a tube in yet a further embodiment.

### DETAILED DESCRIPTION

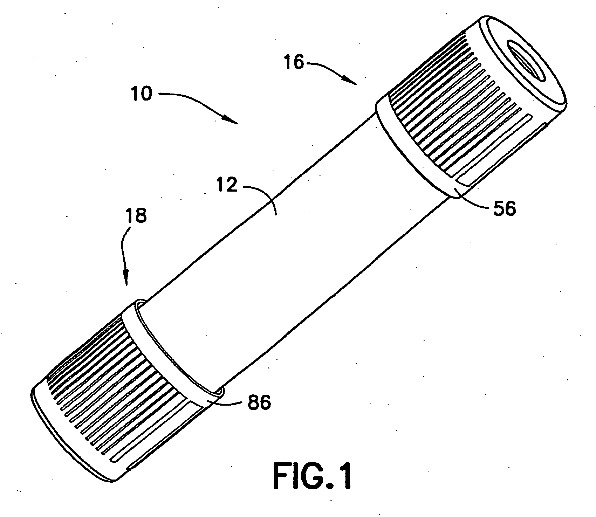

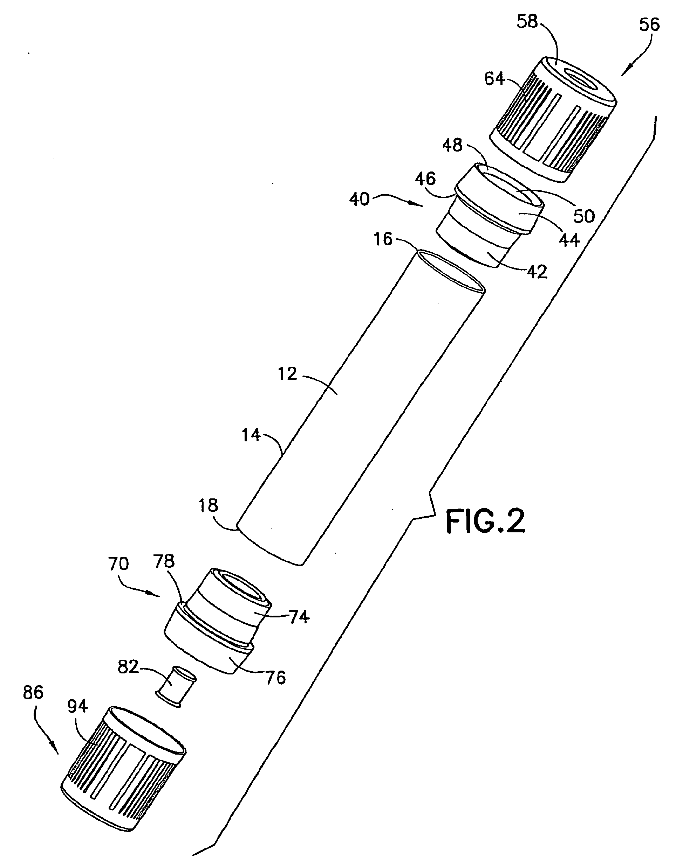

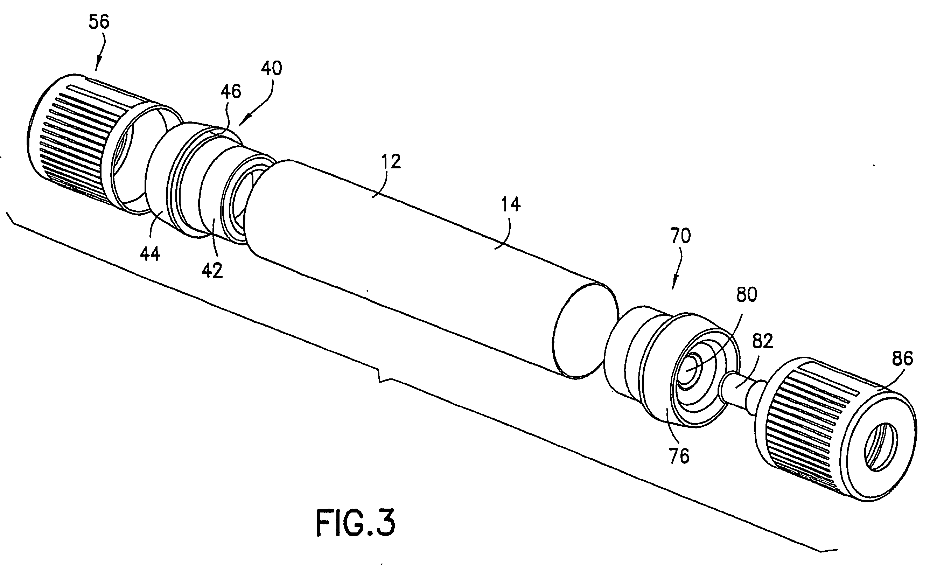

Referring to the drawings in which like reference characters refer to the like parts throughout the several views thereof. FIGS. 1-4 illustrate a specimen collection container in accordance with a preferred embodiment of the present invention and the related features. As shown in FIGS. 1-4, the specimen collection container **10** includes a tube **12**, including a piercible closure **40** at one end and a vent such as hybrid stopper **70** extending from the opposing end thereof.

More particularly, tube **12** includes a generally cylindrical tubular wall **14** extending between a first end **16** at the top thereof and a second end **18** at the bottom thereof, defining an interior chamber **20** of tube **12**. Tube **12** may be made out of any suitable material which is impermeable to liquid, preferably impermeable to gas and liquid, and is desirably made out of glass or molded plastic. Additionally, tube **12** may be constructed in any practical size for obtaining an appropriate biological sample. For example, tube **12** may be of a size similar to conventional large volume tubes, small volume tubes, or microcollection tubes, as is known in the art.

Tube **12** includes a piercible closure **40** sealingly covering first end **16** thereof. Piercible closure **40** is constructed of a suitable material capable of providing the open first end **16** of tube **12** with a liquid-tight seal, and capable of being punctured or pierced with an appropriate medical device, such as a needle cannula, for transfer of a biological sample into tube **12**. Desirably, piercible closure **40** is constructed of an elastomeric material, such as rubber.

Piercible closure **40** may be mated with first end **16** of tube **12** through any mechanism, such as a snap-fit, a friction fit, a threaded connection, or the like. Desirably, piercible closure **40** is constructed of an elastomeric material and is mated with first end **16** in a friction-fit arrangement. For example, piercible closure **40** may include a depending portion **42** extending from a main portion **44**, with depending portion **42** extending within the opening at first end **16** of tube **12** and into interior chamber **20** thereof. Piercible closure **40** includes an annular shoulder **46** which mates with first end **16** of tube **12.** Further, piercible closure **40** has a top surface **48,** which may include a depending recess **50** centrally molded or fabricated thereon. The outer diameter of depending portion **42** of piercible closure **40** is substantially the same as the inner diameter of interior chamber **20** of tube **12**. Also, the outer diameter of main portion **44** at annular shoulder **46** of piercible closure **40** is greater than the inner diameter of interior chamber **20** of tube **12**. In this manner, when piercible closure **40** is removably mated to first end **16** of tube **12** annular shoulder **46** rests on the edge of first end **16,** and a gas-and liquid-tight seal is formed.

A cap element **56** may further be provided and mated with first end **16** of tube **12** about piercible closure **40.** More particularly, cap element **56** may be provided including a front face **58** with an annular skirt **60** depending therefrom. Front face **58** of cap element **56** includes a central opening such as through hole **62** extending therethrough. Cap element **56** is desirably positioned over piercible closure **40** and is engaged with the outer surface of tube **12** at first end **16** thereof, such as through a friction-fit, a snap-fit, a threaded engagement or the like, or may be adhesively affixed or adhered thereto. Cap element **56** may desirably be constructed of a plastic or polymeric material, and desirably includes a series of ribs **64** for providing a tactile surface. Also, cap element **56** may be color-coded, providing the user with an identification of the contents of the tube **12,** or the intended use or intended testing of the contents of the tube **12.**

Tube **12** further includes a vent element at opposing second end **18** thereof. Such a vent element is a physical structure which is adapted for venting of air within interior chamber **20** of tube **12**, while maintaining a closed environment, in particular a liquid-tight environment, within interior chamber **20** of tube **12**. The vent element may be integrally formed with tube **12**, or may be a separate element which is separately attached to tube **12.**

Desirably, such a vent element is provided through a hybrid stopper **70** which includes an opening for accommodating a venting structure therein and maintaining the venting structure in fluid communication with the interior chamber **20** of tube **12**. For example, hybrid stopper **70** may be mated with an open second end **18** through any mechanism, such as a snap-fit, a friction fit, a threaded connection, or the like, such as with piercible closure **40** mated at first end **16**. Desirably, hybrid stopper **70** is constructed of an elastomeric material such as rubber, and includes a depending portion **74** extending from a main portion **76**, with an annular shoulder **78** therebetween. Annular shoulder **78** mates with second end **18** of tube **12** such that depending portion **74** extends within the opening at second end **18** and into interior chamber **20** thereof. As with the piercible closure **40**, the outer diameter of depending portion **74** of hybrid stopper **70** is substantially the same as the inner diameter of interior chamber **20** of tube **12**, and the outer diameter of main portion **76** at annular shoulder **78** of hybrid stopper **70** is greater than the inner diameter of interior chamber **20** of tube **12**. In this manner, when hybrid stopper **70** is removably mated to second end **18** of tube **12** annular shoulder **78** rests on the edge of second end **18**, and a gas- and liquid-tight seal is formed.

Hybrid stopper **70** further includes a central opening **80** extending therethrough between opposing ends thereof. Central opening **80** provides a path for fluid communication between interior chamber **20** of tube **12** and the external environment when hybrid stopper 70 is sealingly positioned over open second end **18**. A venting filter **82** is located within central opening **80**, and is desirably affixed therein, for example through a suitable medical grade adhesive. Venting filter **82** permits air and other gases to flow from within internal chamber **20** to the external environment. Venting filter **82** maybe a two-way vent, in which air is able to flow in both directions within and out of internal chamber **20,** thereby facilitating adjustment in different barometric or ambient pressures. While the venting filter **82** permits air and gas to flow therethrough, it acts as a barrier for liquid flow in either direction. Also, once a liquid specimen such as blood contacts and/or saturates the venting filter **82**, the vent will seal to any further gas flow therethrough. As such, no air will be able to flow in or out of the vent, even once the liquid specimen has been removed. This characteristic decreases the chances of contaminants being able to enter the tube and contact a liquid sample contained within the interior chamber of the tube. However, this characteristic necessitates care on the part of the phlebotomist in that the liquid sample should not contact the venting filter **82** prematurely.

Venting filter **82** may be made out of any suitable material which is permeable to gases (in particular air) and impermeable to liquids (in particular blood), and is desirably constructed of a polymeric material such as high density polyethylene or high density polypropylene. A particularly useful material is available from Porex Porous Products.

As with first end **16** of tube **12,** a cap element **86** may further be provided and mated with second end **18** of tube **12** about hybrid stopper **70.** More particularly, cap element **86** may be provided including a front face **88** with an annular skirt **90** depending therefrom. Front face **88** of cap element **86** includes a central opening such as through-hole **92** extending therethrough. Cap element **86** is desirably positioned over hybrid stopper **70** and is engaged with the outer surface of tube **12** at second end **18** thereof, such as through a friction-fit, a snap-fit, a threaded engagement or the like, or may be adhesively affixed or adhered thereto. Cap element **86** may desirably be constructed of a plastic or polymeric material, and desirably includes a series of ribs **94** for providing a tactile surface. Also, cap element **86** may be color-coded, desirably with the same color coding as cap element **56** at first end **16** of tube **12.**

It is also contemplated that the arrangement of the components of the hybrid stopper may be reversed. For example, the venting filter may be constructed of a material which can provide an effective liquid-tight seal directly within the tubular wall **14** at open bottom end **18** of tube **12**, without the need for any separate elastomeric material sealing against the tubular wall **14**. In such an arrangement, a separate piercible insert member may be provided through the venting filter, for providing a mechanism for piercible access. Such an embodiment effectively provides for an annular venting filter around a piercible insert member. It is further contemplated that in such an embodiment, such a hybrid stopper including a piercible insert within an annular filter member forms a closure at one end of the tube, while the opposing end of the tube may be closed off through a separate stopper, or may be formed as a closed-ended tube. Such an alternate hybrid stopper may provide both piercible access to the interior chamber **20**, as well as the venting element for effective displacement of air during sampling and transfer of a sample into tube **12.**

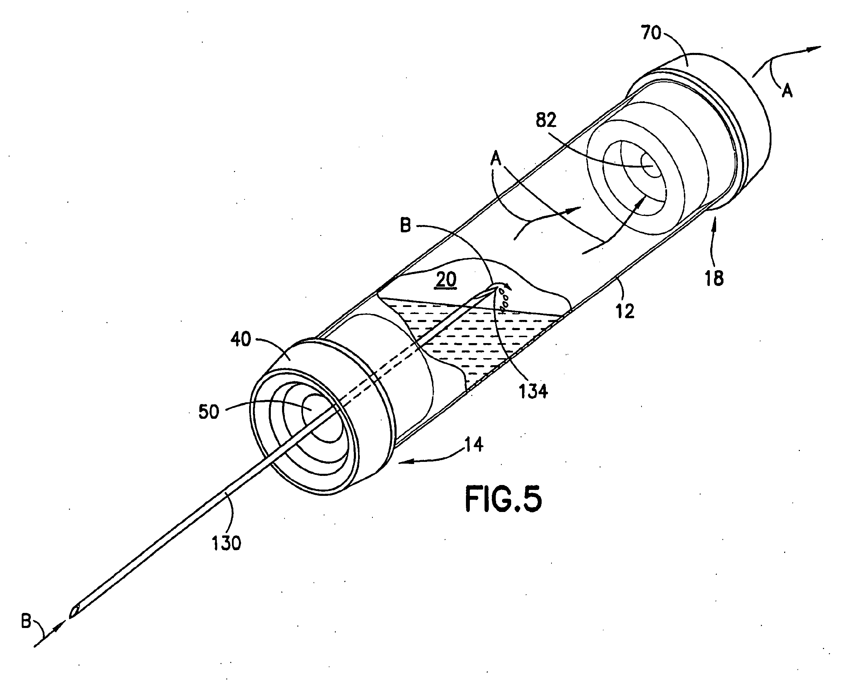

Use of the specimen collection container **10** in connection with drawing a blood sample from a patient is shown in FIG. 5, with cap elements **56** and **86** deleted from FIG. 5 for clarity. In use, a double-ended phlebotomy-type needle such as needle **130** having an intravenous puncture tip (not seen) and a non-patient puncture tip **134** may be connected with a standard needle holder (not shown) and inserted into a patient's blood vessel such as a vein, as is known in the art. In typical blood draw procedures, at this point once the vein is accessed, the non-patient needle **134** is used to pierce a stopper of an evacuated collection tube, and the negative pressure established by the vacuum within such a collection tube draws the blood sample from the patient's vein through the needle cannula and into the tube. As discussed above, however, such procedures can be problematic, with the vacuum oftentimes causing the vein to collapse, particularly for patient's with weakened blood vessels such as infants or the elderly. Also, the exact vacuum within such collection tubes can be difficult to control over time, resulting in a loss of negative pressure within the evacuated tube and an ineffective blood draw.

In the present invention, the non-patient puncture tip **134** of the needle **130** is used to puncture piercible closure **40** through recess **50** of piercible closure **40** at first end **16** of tube **12.** As illustrated in FIG. 5, blood travels through needle **130** as shown through arrows B, and enters the interior chamber **20** of tube **12** by way of the patient's blood pressure. More particularly, the blood within the patient's vein is pumping through the vein at physiological pressure, which is greater than normal atmospheric or ambient pressure. Since the interior chamber **20** of tube **12** is vented to the external environment, the air pressure within interior chamber **20** is ambient pressure. When the blood within the patient's vein is in fluid contact with the interior chamber such as when the non-patient puncture tip **134** pierces piercible closure **40**, the higher pressure of the patient's vein forces the blood into the lower ambient pressure interior chamber **20**. As such, the blood transfer occurs based on the patient's venous pressure.

During such transfer of blood into the interior chamber **20,** it is necessary to displace the air within the interior chamber **20** in order to prevent the pressure therein from building to a pressure which would be above the venous pressure and therefore prevent further blood flow into interior chamber **20.** Accordingly, during such blood flow, the air, and any other gases present within interior chamber **20**, must be displaced from within interior chamber **20**. This displacement occurs by venting such air from within interior chamber **20** through venting filter **82** at second end **18** of tube **12**, as shown through the directional arrows A. Accordingly, as blood flows into interior chamber **20** through needle **130** as depicted in directional arrows B, the air within interior chamber **20** is correspondingly displaced and passes through venting filter **82** as shown through arrows A. When a sufficient amount of blood is collected within the interior chamber **20** of tube **12**, the tube **12** can be removed from non-patient puncture tip **134**, thereby stopping the flow of blood into the interior chamber **20.**

Also, the nature of venting filter **82** provides a further mechanism for stopping the blood flow. In particular, as noted above, venting filter **82** may be constructed of a material which becomes gas impermeable once it is contacted by blood. During a blood sampling procedure, the container **10** is typically inverted so as to push the piercible closure **40** onto non-patient puncture tip **134**. In this position, the second end **18** of tube **12** with venting filter **82** contained within hybrid stopper **70** is positioned at the top of the container **10**. Accordingly, during sampling, the blood will rise within the interior chamber **20** of the tube **12** until it is full, at which point the blood will contact the venting filter **82**. Such contact will cause venting filter **82** to become gas impermeable, thereby preventing any further blood to enter interior chamber **20** since no further air can be displaced therefrom. Also, such feature serves to protect the blood sample within the tube **12** when a two-way filter is used, since no further air will be able to enter into the interior chamber through the now-sealed venting filter **82** either.

If desired, collection container **10** may be provided with an additive for imparting desired properties to the sample collected therein. For example, one or more additives such as reagents, preservatives, anticoagulants, procoagulants, clot activators, and other known additives may be provided within the interior chamber **20** of tube **12** to provide for a desired effect on the sample. Desirably, such additives are provided in dry form such as a powder or pellet, so as not to effect the gas permeability of venting filter **82.**

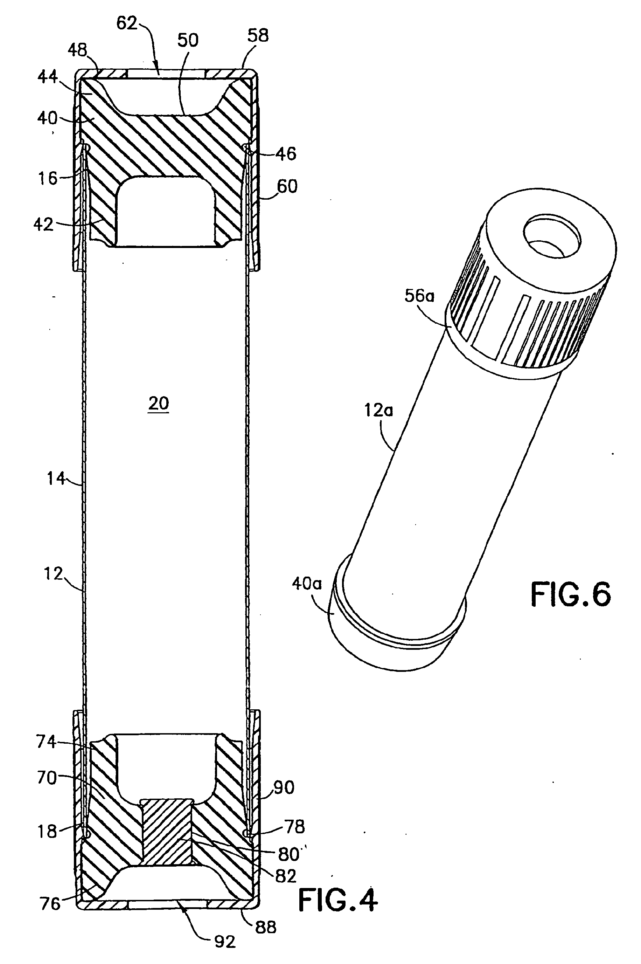

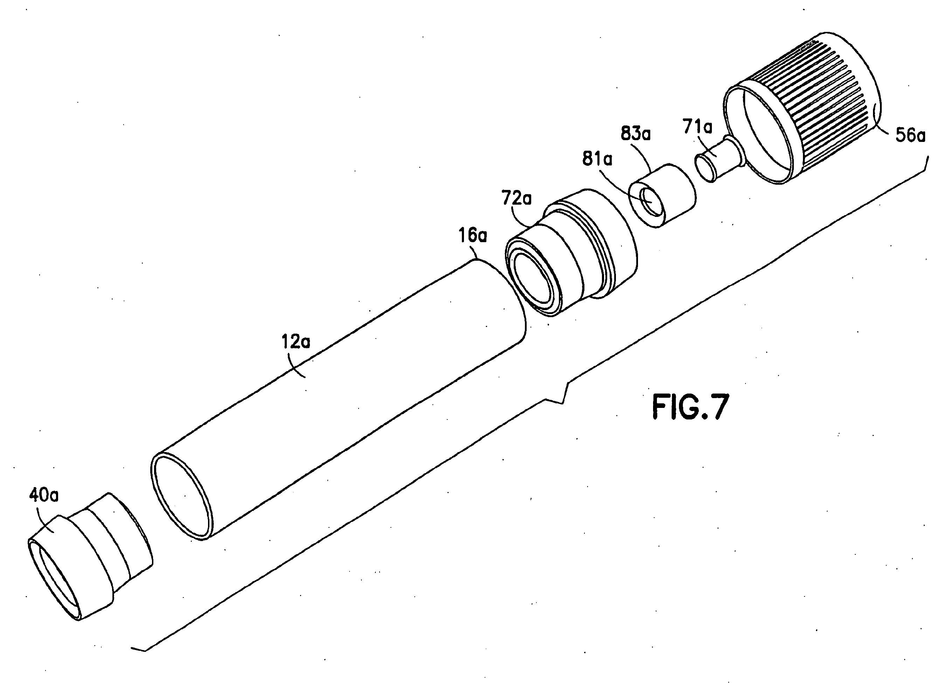

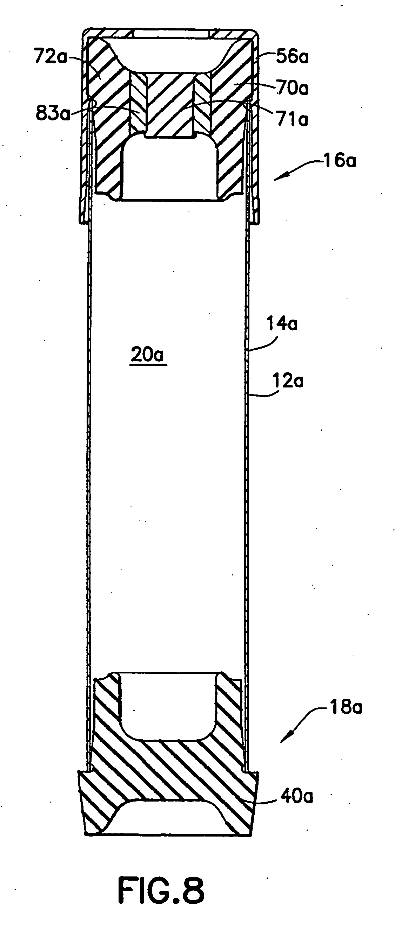

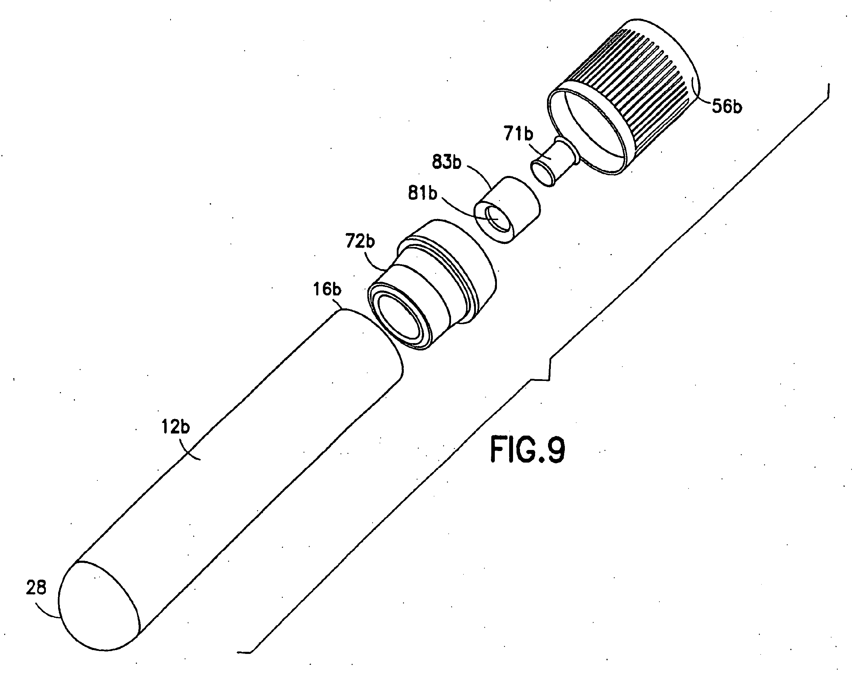

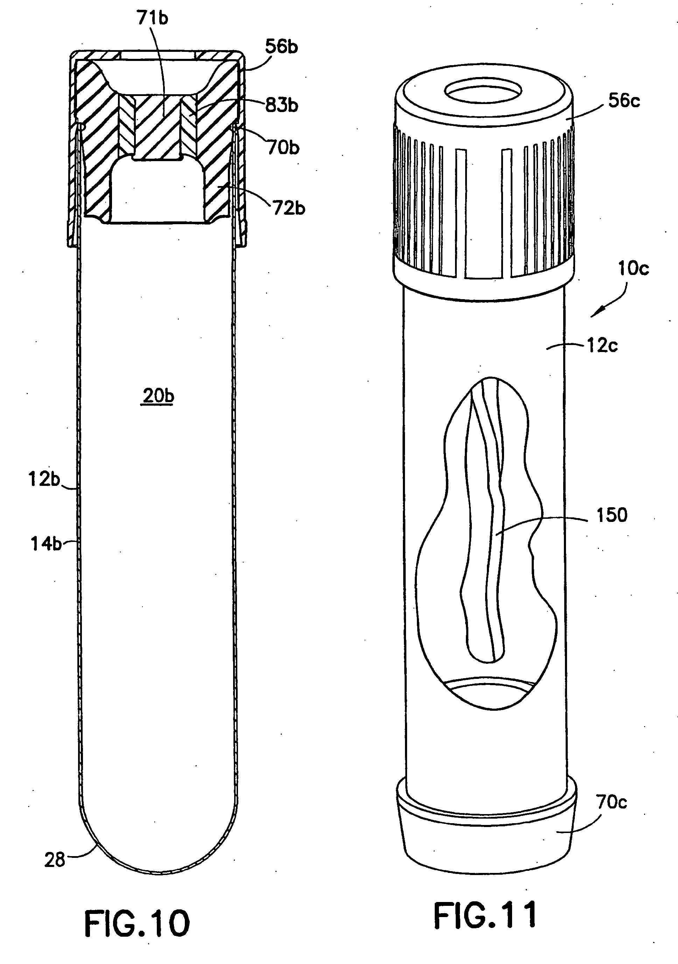

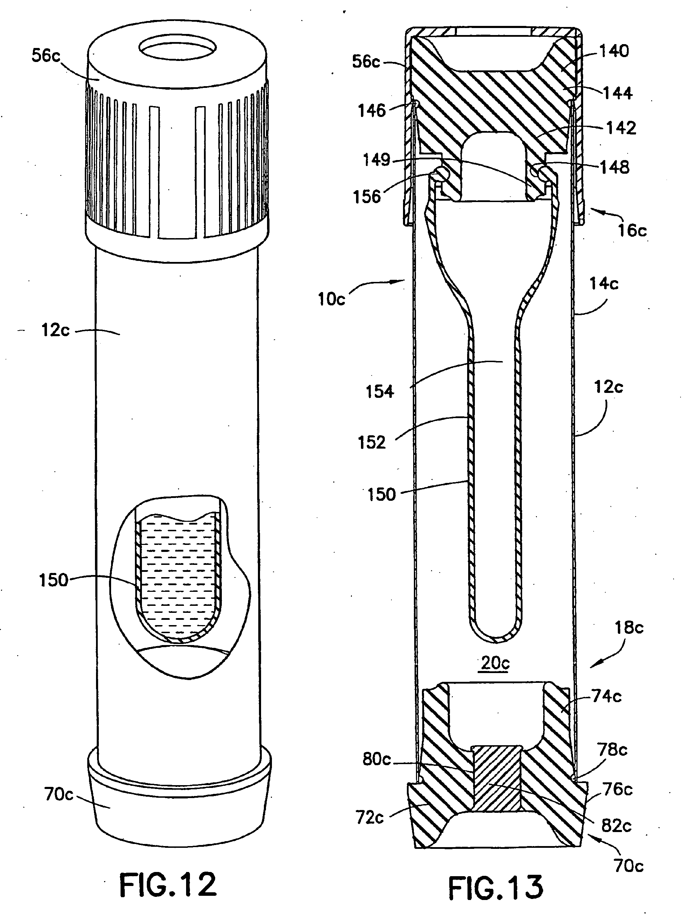

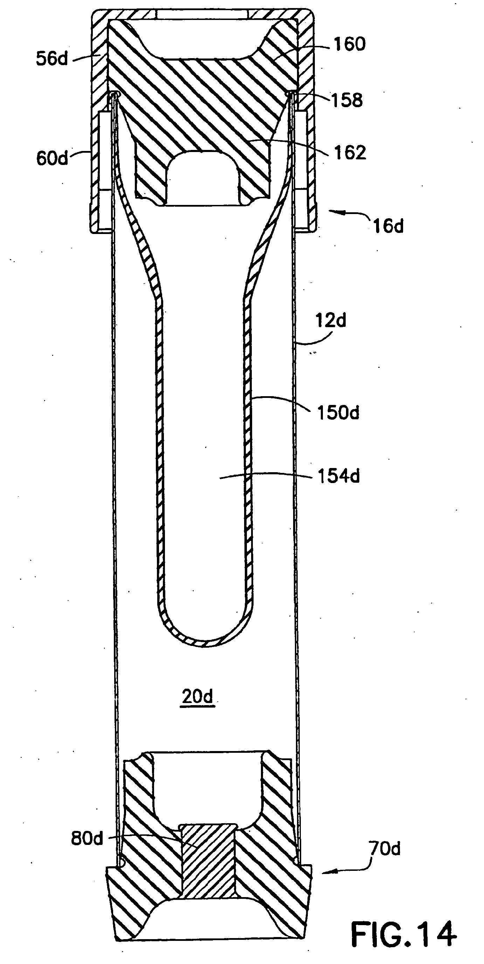

FIGS. 6-14 depict further embodiments of the invention that include many components which are substantially identical to the components of FIGS. 1-4. Accordingly, similar components performing similar functions will be numbered identically to those components of FIGS. 1-4, except that a suffix "a" will be used to identify those similar components in FIGS. 6-8, a suffix "b" will be used to identify those similar components in FIGS. 9-10, a suffix "c" will be used to identify those similar components in FIGS. 11-13, and a suffix "d" will be used to identify those similar components in FIG. 14.

FIGS. 6-8 depict a slight variation on the embodiment of the specimen collection container **10** shown in FIGS 1-4. In essence, in the embodiment of FIGS. 6-8, the collection container is inverted from that of the previously described embodiment in FIGS. 1-4, such that the hybrid stopper **70a** is positioned at the first end **16a** of tube **12a.** A separate closure **40a** is provided at opposing second end **18a.** While closure **40a** in this embodiment is desirably an elastomeric material and is similar to piercible closure **40** described above, closure **40a** is not necessarily intended to be pierced as in the previous embodiment. Instead, in the embodiment of FIGS. 68, both the vent means and the means for accessing the interior portion of the collection container are desirably provided through the hybrid stopper **70a.** As such, access to interior chamber **20a** may be provided through hybrid stopper **70a** provided at first end **16a.**

More particularly, hybrid stopper **70a** may include a specified portion which is adapted for piercing with a needle and a portion which is adapted for venting air therethrough. This may be achieved by providing hybrid stopper **70a** with multiple components including elastomeric body **72a** having a central bore extending therethrough and an annular venting filter **83a** positioned and maintained within the central bore through elastomeric body **72a.** Annular venting filter **83a** includes a central bore **81a,** and an elastomeric plug **71a** is further provided and maintained within central bore **81a**. In practice of the embodiment shown in FIGS. 6-8, the elastomeric plug **71a** is pierced with the non-patient end of a needle for use thereof. Elastomeric plug **71a** should be sufficiently maintained within central bore **81a** so as not to be displaced during piercing with a needle in such a procedure. This may be accomplished, for example, through friction fit, through the design profile of elastomeric plug **71a** interfitting within central bore **81a**, or through the use of an adhesive. As blood enters interior chamber **20a** of tube **12a,** displaced air is forced out of interior chamber **20a** through venting filter **83a** located within hybrid stopper **70a.** Alternatively, elastomeric body **72a** of hybrid stopper **70a** may be directly pierced with a needle. Cap element **56a** may also be provided over first end **16a** of tube **12a,** thereby encompassing and containing hybrid stopper **70a** therein. A separate cap element (not shown in FIGS. 6-8) may be provided over closure **40a** at second end **18a** if desired. .

In yet a further embodiment, the venting filter may be constructed of a material which, in addition to providing the venting and sealing features as described above, is also capable of being pierced with a needle and capable of re-sealing after removal of the needle therefrom. With such an embodiment, the venting filter may be positioned centrally within the stopper element, or may comprise the entire stopper element sealing the end of the tube.

As shown in FIGS. 9-10, a further embodiment of a specimen collection container in accordance with the present invention includes a non-evacuated collection tube **12b** having a tubular wall **14b** extending between an open top first end **16b** and a closed bottom second end **28** defining an interior chamber **20b**. In such an embodiment, hybrid stopper **70b** including venting filter **83b** within elastomeric body **72b** is provided at the open top first end **16b** of tube **12b**, as with the embodiment described in FIGS. 6-8. Such an embodiment provides for a similar collection container as in the embodiment of FIGS. 6-8 and is used in a similar manner, with the exception that the closed second end **28** of tube **12b** provides for an entirely closed environment, and eliminates the potential for leaks or spilling through a separate closure such as closure **40a** shown in FIGS. 6-8.

FIGS. 11-13 illustrate a further embodiment, which includes a secondary sample container contained within the primary container provided through the specimen collection container, such as an expandable sample bag within the collection container. More particularly, specimen collection container **10c** includes a tube **12c,** having a closure **140,** a hybrid stopper **70c,** and an expandable bag **150.** Tube **12c** includes a tubular wall **14c** extending between a first end **16c** and a second end **18c,** which defines an interior chamber **20c** in a similar manner as in the embodiment of FIGS. 1-4. Further, collection container **10c** includes a hybrid stopper **70c** desirably constructed of an elastomeric body **72c** with a depending portion **74c** extending within second end **18c** of tube **12c,** and with annular shoulder **78c** resting upon second end **18c.** Hybrid stopper **70c** also has a central opening **80c** medially located with a venting filter **82c** located within central opening **80c,** as described above.

In the embodiment of FIGS. 11-13, the first end **16c** of tube **12c** is desirably sealed through closure **140,** which may further include a cap element **56c** which may be similar in design and construction as that described in the previous emboidments. Closure **140** is mated with the first end **16c** of tube **12c** through any mechanism, such as a snap-fit, a friction fit, a threaded connection, or the like, with cap element **56c** positioned thereover. Closure **140** includes a depending portion **142** extending from a main body portion **144,** with depending portion **142** extending within the first end **16c** of tube **12c.** Closure **140** includes an annular shoulder **146** which mates with first end **16c** of tube **12c.** The outer diameter of depending portion **142** of piercible closure **140** is substantially the same as the inner diameter of interior chamber **20c** of tube **12c.** Also, the outer diameter of main portion **144** at annular shoulder **146** ofpiercible closure **140** is greater than the inner diameter of interior chamber **20c** of tube **12c.** In this manner, when piercible closure **140** is removably mated to first end **16c** of tube **12c** annular shoulder **146** rests on the edge of first end **16c,** and a gas- and liquid-tight seal is formed. Further, depending portion **142** includes an annular groove **148** extending about a perimeter thereof contained within interior chamber **20c** when closure **140** is mated with tube **12c**. This annular groove **148** is desirably formed in a narrowed neck portion **149,** which has an outer diameter smaller than that of the depending portion **142.**

Collection container **10c** also includes further structure maintained within interior chamber **20c** for holding the blood or biological sample therein. For example, an internal container such as a balloon or an expandable bag **150** may be provided extending into interior chamber **20c**. Such expandable bag **150** is adapted for containing the blood or other biological sample entirely therein during the collection procedure, and in effect provides for a secondary container structure for collection of the sample. Expandable bag **150** has a bag wall **152** defining an interior bag chamber **154**. Expandable bag **150** may be constructed of any material capable of containing a biological sample and capable of expanding upon exposure to venous pressure. Desirably, expandable bag **150** is a polymeric material which is compatible with blood, has minimal rigidity, and can expand under venous pressure. Examples of useful materials include, but are not limited to, polyethylene or polyvinyl chloride.

Expandable bag extends from the first end **16c** within internal chamber **20c** of tube **12c.** Desirably, expandable bag is mated with closure **140.** For example, expandable bag **150** may include an annular ring **156** which is matable with the annular groove **148** on the depending portion **142** of closure **140**. In such instances, it may be desirable to include a non-puncturable material at the top portion of expandable bag **150** which is adjacent first end **16c,** so as to prevent expandable bag **150** from being punctured by a needle during a sample procedure. By providing expandable bag **150** attached with closure **140**, expandable bag **150** with a sample contained therein can be removed from tube **12c** along with closure **140** after a sampling procedure in order to access the sample contained within expandable bag **150.**

Prior to use, expandable bag **150** is collapsed, with no air contained within interior chamber **154** of expandable bag **150,** as shown in FIG. 11. During use, a non-patient puncture tip of a needle can piercably engage closure **140**. Such piercing establishes a path for fluid flow through the needle into interior chamber **154** of expandable bag **150**. Blood can then enter and fill interior bag chamber **154** of expandable bag **150** based upon the venous pressure, and expandable bag **150** will then expand based upon the blood entering expandable bag **150** under the venous pressure. Expansion of expandable bag **150** will force any air which is present inside interior chamber **20c** of tube **12c** out to ambient through venting filter **82c** at second end **18c** of tube **12c**. This further embodiment significantly minimizes operator error in that, assuming complete structural integrity of the expandable bag **150** is maintained, blood collected within interior bag chamber **154** of expandable bag **150** will not prematurely contact and seal off venting filter **82c** at second end **18c** of tube **12c.**

FIG. 14 depicts a slight variation of the embodiment in FIGS. 11-13, in which the top end of the expandable bag **150d** includes an annular lip portion **158,** which is folded over the top edge of first top end **16d** of tube **12d** (as opposed to the annular ring **156** within the annular groove **148)** and is secured in place through frictional engagement on the exterior of first end **16d** of tube **12d** with annular skirt **60d** of cap element **56d** and on the interior of first end **16d** of tube **12d** with closure **160**. In this embodiment, the depending portion **162** of closure **160** is substantially smaller in diameter than the inner diameter of tube **12d** at first end **16d** such that the expandable bag **150d** can extend between closure **160** and tube **12d.**

While the present invention is satisfied by embodiments in many different forms, there is shown in the figures and described herein in detail, the preferred embodiments of the invention, with the understanding that the present disclosure is to be considered as exemplary of the principles of the invention and is not intended to limit the invention to the embodiments illustrated. Various other embodiments will be apparent to and readily made by those skilled in the art without departing from the scope of the invention. The scope of the invention will be measured by the appended claims and their equivalents.

## Claims

1. A specimen collection container comprising:
a non-evacuated collection tube (12) including a tubular wall (14) extending between an open top end (16) and a bottom end (18) defining an interior chamber (20),
a pierceable closure (40;70a;70b) sealing said open top end of said collection tube, and
a vent (80,82;83a;83b) adapted for displacement of air from said interior chamber (20) of said collection tube to an exterior of said collection tube during collection of a liquid sample within said interior chamber of said collection tube, wherein
said vent (80,82;83a;83b) is adapted to seal upon contact with said liquid sample so as to prevent said displacement of air,
wherein said bottom end (18) of said collection container (12) is open, said specimen collection container further comprising a second closure (70) sealing said open bottom end of said collection tube, **characterized in that** said vent (83a;83b) extends through at least a portion of said second closure (70).

2. A specimen collection container as in claim 1, wherein said vent (80,82) is adapted for maintaining said interior chamber at ambient pressure prior to collection of a sample within said interior chamber (20).

3. A specimen collection container as in claim 2, wherein said vent (80,82) comprises a two-way vent.

4. A specimen collection container as in claim 1, wherein said pierceable closure (40) is removably attached to said open top end (16) of said collection tube and said second closure (70) is removably attached to said bottom end of said collection tube.

5. A specimen collection container as in claim 1, wherein said second closure (70) comprises a hybrid stopper removably attachable to said open bottom end.

6. A specimen collection container as in claim 5, wherein said hybrid stopper is comprised of an elastomeric material (80) and a venting filter (82).

7. A specimen collection container as in claim 6, wherein said venting filter (82) comprises a material selected from the group consisting of high density polyethylene and high density polypropylene.

8. A specimen collection container as in claim 1, wherein said vent (83a;83b) extends through at least a portion of said pierceable closure (70a;70b).

9. A specimen collection container as in claim 8, wherein said pierceable closure (70a;70b) comprises a hybrid stopper removably attachable to said open top end.

10. A specimen collection container as in claim 9, wherein said hybrid stopper is comprised of an elastomeric material (72a) and a venting filter (82;83a;83b).

11. A specimen collection container as in claim 10, wherein said venting filter comprises a material selected from the group consisting of high density polyethylene and high density polypropylene.

12. A specimen collection container as in claim 1, further comprising an internal container (150) positioned within the interior chamber (20c) of said collection tube, said internal container establishing a closed environment for containment of a liquid sample.

13. A specimen collection container as in claim 12, wherein said internal container (150) further comprises an expandable bag.

14. A specimen collection container as in claim 13, wherein the expandable bag is in sealed engagement with said open top end (16c) of said collection tube (12c).

15. A specimen collection container as in claim 13, wherein the expandable bag is in sealed engagement with said pierceable closure (140).

## Patentansprüche

1. Probensammelbehälter mit:
einem nicht evakuierten Sammelröhrchen (12) mit einer sich zwischen einem offenen oberen Ende (16) und einem unteren Ende (18) erstreckenden rohrförmigen Wand (14), die eine Innenkammer (20) bildet,
einem durchstechbaren Verschluss (40; 70a; 70b), der das offene obere Ende des Sammelröhrchens dicht abschließt, und
einer Lüftung (80, 82; 83a; 83b), die geeignet ist, während des Sammelns einer Flüssigkeitsprobe in der Innenkammer des Sammelröhrchens Luft aus der Innenkammer (20) des Sammelröhrchens aus dem Sammelröhrchen heraus zu verdrängen,
wobei die Lüftung (80, 82; 83a; 83b) geeignet ist, bei Kontakt mit der Flüssigkeitsprobe dicht abzuschließen, um das Verdrängen von Luft zu verhindern,
wobei das untere Ende (18) des Sammelröhrchens (12) offen ist, wobei der Probensammelbehälter ferner einen zweiten Verschluss (70) aufweist, welcher das offene untere Ende des Sammelröhrchens dicht abschließt,
**dadurch gekennzeichnet, dass**
sich die Lüftung (83a; 83b) durch zumindest einen Teil des zweiten Verschlusses (70) erstreckt.

2. Probensammelbehälter nach Anspruch 1, bei welchem die Lüftung (80, 82) geeignet ist, die Innenkammer vor dem Sammeln einer Probe in der Innenkammer (20) auf Umgebungsdruck zu halten.

3. Probensammelbehälter nach Anspruch 2, bei welchem die Lüftung (80, 82) eine Zwei-Wege-Lüftung aufweist.

4. Probensammelbehälter nach Anspruch 1, bei welchem der durchstechbare Verschluss (40) abnehmbar an dem offenen oberen Ende (16) des Sammelröhrchens angebracht ist, und der zweite Verschluss (70) abnehmbar an dem unteren Ende des Sammelröhrchens angebracht ist.

5. Probensammelbehälter nach Anspruch 1, bei welchem der zweite Verschluss (70) einen Hybridstopfen aufweist, der abnehmbar an dem offenen unteren Ende anbringbar ist.

6. Probensammelbehälter nach Anspruch 5, bei welchem der Hybridstopfen aus einem Elastomermaterial (80) und einem Lüftungsfilter (82) besteht.

7. Probensammelbehälter nach Anspruch 6, bei welchem das Lüftungsfilter (82) ein Material aufweist, das aus der Gruppe bestehend aus hochdichtem Polyethylen und hochdichtem Polypropylen gewählt ist.

8. Probensammelbehälter nach Anspruch 1, bei welchem die Lüftung (83a; 83b) sich durch zumindest einen Teil des durchstechbaren Verschlusses (70a; 70b) erstreckt.

9. Probensammelbehälter nach Anspruch 8, bei welchem der durchstechbare Verschluss (70a; 70b) einen Hybridstopfen aufweist, der abnehmbar an dem offenen oberen Ende anbringbar ist.

10. Probensammelbehälter nach Anspruch 9, bei welchem der Hybridstopfen aus einem Elastomermaterial (72a) und einem Lüftungsfilter (82; 83a; 83b) besteht.

11. Probensammelbehälter nach Anspruch 10, bei welchem das Lüftungsfilter ein Material aufweist, das aus der Gruppe bestehend aus hochdichtem Polyethylen und hochdichtem Polypropylen gewählt ist.

12. Probensammelbehälter nach Anspruch 1, ferner mit einem in der Innenkammer (20c) des Sammelröhrchens angeordneten Innenbehälter (150), wobei der Innenbehälter eine geschlossene Umgebung für das Aufnehmen einer Flüssigkeitsprobe bildet.

13. Probensammelbehälter nach Anspruch 12, bei welchem der Innenbehälter (150) ferner einen dehnbaren Beutel enthält.

14. Probensammelbehälter nach Anspruch 13, bei welchem der dehnbare Beutel sich in dichtem Eingriff mit dem offenen oberen Ende (16c) des Sammelröhrchens (12c) befindet.

15. Probensammelbehälter nach Anspruch 13, bei welchem der dehnbare Beutel sich in dichtem Eingriff mit dem durchstechbaren Verschluss (140) befindet.

## Revendications

1. Récipient de recueil d'échantillon, comprenant :
un tube de recueil dans lequel on n'a pas fait le vide (12) comprenant une paroi tubulaire (14) s'étendant entre une extrémité supérieure ouverte (16) et une extrémité inférieure (18) définissant une chambre intérieure (20),
une fermeture perforable (40 ; 70a ; 70b) obturant ladite extrémité supérieure ouverte dudit tube de recueil, et
un évent (80, 82 ; 83a ; 83b) adapté pour déplacer de l'air de ladite chambre intérieure (20) dudit tube de recueil à l'extérieur dudit tube de recueil pendant le recueil d'un échantillon de liquide dans ladite chambre intérieure dudit tube de recueil, dans lequel
ledit évent (80, 82 ; 83a, 83b) est adapté pour se fermer par contact avec ledit échantillon liquide de façon à empêcher ledit déplacement d'air,
dans lequel ladite extrémité inférieure (18) dudit conteneur de recueil (12) est ouverte, ledit conteneur de recueil d'échantillon comprenant en outre une deuxième fermeture (70) obturant ladite extrémité inférieure ouverte dudit tube de recueil, **caractérisé en ce que** ledit évent (83a, 83b) s'étend dans au moins une partie de ladite deuxième fermeture (70).

2. Récipient de recueil d'échantillon selon la revendication 1, dans lequel ledit évent (80, 82) est adapté pour maintenir ladite chambre intérieure à une pression ambiante avant de recueillir un échantillon dans ladite chambre intérieure (20).

3. Conteneur de recueil d'échantillon selon la revendication 2, dans lequel ledit évent (80, 82) comprend un évent bilatéral.

4. Récipient de recueil d'échantillon selon la revendication 1, dans lequel ladite fermeture perforable (40) est fixée de façon amovible à ladite extrémité supérieure ouverte (16) dudit tube de recueil et ladite deuxième fermeture (70) est fixée de façon amovible à ladite extrémité inférieure dudit tube de recueil.

5. Récipient de recueil d'échantillon selon la revendication 1, dans lequel ladite deuxième fermeture (70) comprend un arrêt hybride pouvant être fixé de façon amovible à ladite extrémité inférieure ouverte.

6. Récipient de recueil d'échantillon selon la revendication 5, dans lequel ledit arrêt hybride est constitué d'un matériau élastomère (80) et d'un filtre d'aération (82).

7. Récipient de recueil d'échantillon selon la revendication 6, dans lequel ledit filtre d'aération (82) comprend un matériau choisi dans le groupe comprenant le polyéthylène haute densité et le polypropylène haute densité.

8. Récipient de recueil d'échantillon selon la revendication 1, dans lequel ledit évent (83a ; 83b) s'étend dans au moins une partie de ladite fermeture perforable (70a ; 70b).

9. Récipient de recueil d'échantillon selon la revendication 8, dans lequel ladite fermeture perforable (70a ; 70b) comprend un arrêt hydride pouvant être fixé de façon amovible à ladite extrémité supérieure ouverte.

10. Récipient de recueil d'échantillon selon la revendication 9, dans lequel ledit arrêt hybride est constitué d'un matériau élastomère (72a) et d'un filtre d'aération (82 ; 83a ; 83b).

11. Récipient de recueil d'échantillon selon la revendication 10, dans lequel ledit filtre d'aération comprend un matériau choisi dans le groupe comprenant le polyéthylène haute densité et le polypropylène haute densité.

12. Récipient de recueil d'échantillon selon la revendication 1, comprenant en outre un récipient interne (150) placé dans la chambre intérieure (20c) dudit tube de recueil, ledit récipient interne établissant un environnement fermé pour contenir un échantillon liquide.

13. Récipient de recueil d'échantillon selon la revendication 12, dans lequel ledit conteneur interne (150) comprend en outre une poche expansible.

14. Récipient de recueil d'échantillon selon la revendication 13, dans lequel ladite poche expansible est en mise en prise scellée avec ladite extrémité supérieure ouverte (16c) dudit tube de recueil (12c).

15. Récipient de recueil d'échantillon selon la revendication 13, dans lequel la poche expansible est en mise en prise scellée avec ladite fermeture perforable (140).
